Europäisches Patentamt

European Patent Office

Office européen des brevets

(19)

(11) Publication number: **0 505 631 A1**

## EUROPEAN PATENT APPLICATION

(12)

(21) Application number: **91302806.4**

(22) Date of filing: **28.03.91**

(51) Int. Cl.⁵: **A61B 17/16, A61M 19/00**

(43) Date of publication of application:
**30.09.92 Bulletin 92/40**

(84) Designated Contracting States:
**AT BE CH DE DK ES FR GB GR IT LI LU NL SE**

(71) Applicant: **Hewitt, John F.**
**5102 East Terry**
**Scottsdale, Arizona 85254(US)**

(72) Inventor: **Hewitt, John F.**
**5102 East Terry**
**Scottsdale, Arizona 85254(US)**

(74) Representative: **Coxon, Philip et al**
**Eric Potter & Clarkson St. Mary's Court St.**
**Mary's Gate**
**Nottingham NG1 1LE(GB)**

(54) **Intraosseous entry device.**

(57) An intraosseous entry device (10) for facilitating the administration of local anesthetic during medical and dental procedures. The device creates a temporary ingress port through the associated gingiva, periosteum and cortical bone into the cancellous bone for delivery of a local anesthetic solution to a predesignated situs. Profound, painless, and immediate anesthesia is thus obtainable for teeth and other structures without creating generalized anesthesia to surrounding soft tissues such as lip, tongue and nostrils. The device comprising an elongated cylinder (12) having a sharp specifically beveled metal face (16) which is embedded within a shank member (18) having a barrier control (20) associated therewith and is driven in response to the driving action of a low speed rotary drill.(27)

FIG 2

INTRODUCTION

The present invention relates generally to means and methods to facilitate the administration of local anesthesia during medical and dental procedures and more particularly to a novel and unique device and method of using same which enables an anesthetic to be quickly and easily delivered to the site of choice without damaging surrounding bone structure or traumatizing the central nervous system.

BACKGROUND OF THE INVENTION

Throughout the history of medical and dental practice, a critical problem facing the practitioner involves the management of pain during certain medical and dental procedures. A variety of methods and apparatus have been heretofor proposed for use in providing local anesthesia for such procedures. These methods and apparatus all have disadvantages either because they are difficult for the practitioner to perform or because they provide a painful, emotionally, traumatic experience for the patient. The problem is especially significant in the practice of dentistry. Health regulations regarding infection control efforts in dentistry are focused on eliminating or minimizing the risk of infection to dental personnel which has heretofor resulted from sharp stick injuries, and from the re-use of devices and needles.

Local anesthesia techniques in current use have certain inherent problems which require special attention before pain management can be perfected. For example, the so-called "infiltration" method, in which a local anesthetic solution is injected into adjoining soft tissue and allowed to disperse around and migrate to the area being treated, suffers from the fact that it frequently takes too long to take effect and is followed by extended period of numbness which inevitably exceeds the period of need. Further, such injections often cause discomfort, ballooning of tissue, the necessity for additional injections to complete work on the desired area, and a potential danger to other vital tissue centers such as the pterygoid plexus.

Another technique for local anesthesia is the "regional block" method, that is, the procedure in which, local anesthetic solution is injected around a nerve trunk where it enters the bone thereby anesthetizing all of the areas served by that trunk. This method gives rise to numerous problems such as, difficulty in locating the nerve trunk, the high level of pain created for most people from the forcible invasion of a nerve the long delay before effect is obtained, the limitation of use to one area per treatment, and the danger in puncturing other areas such as the pterygoid plexus.

In the intraligamentary method commonly used in dental treatment, the anesthetic solution is injected into the peridontal ligament surrounding the tooth to be treated often under considerable pressure. Problems associated with this method include hydraulic effect on the tooth when intense pressure is utilized; post-operative pain; possible inoculation of the gingival crevice with bacteria, which can cause particular problems with heart valve patients; the attainment of a variable and non-predictable level of anesthesia; and an extensive armamentarium of special equipment required to practice the procedure.

Surgical burrs and drills are likewise unsatisfactory alternatives with several disadvantages including cost and repeated need for sterilization and the thermal or mechanical trauma to the bone resulting from their use. For instance, when a given surgical site reaches temperatures in excess of 43oC, the excessive heat can cause protein coagulation and lead to the formation of necrotic bone. Therefore, it is an important object of the present invention to provide means and methods for facilitating the delivery of local anesthesia to a preselected site in a manner which will substantially mitigate and/or eliminate the above defined problems and disadvantages associated with the currently employed procedures to deliver a local anesthetic into specific areas of the body so often required in dental practice.

BRIEF SUMMARY OF THE INVENTION

The present invention relates to a device for facilitating the delivery of local anesthetic solution into and through human osseous structures.

More particularly, the present invention relates to an intraosseous entry device especially designed to create, without damage or erosion to either bone or tissue, temporary ingress to a preselected site for delivery of anesthetic. The device, when disposed in operative coaction with a low speed rotary driving means or drill, institutes a boring-like operation in which the bone adjacent to the rotating blade is compressed sidewise along the opening created thereby without damage to any part thereof. As is known, such compression of bone structure does not register on human pain sensors (as does abrasive substance removal) and is therefore essentially painless.

In one practice of the present invention the device of the present invention comprises a solid cylindrical metal member having a elongated body portion extending between a proximal and a distal end, and a beveled solid face or tip disposed in said distal end; a tubular shank member disposed in circumscription about said elongated body portion and extending between said proximal end of said metal member to a circular barrier member disposed thereabout and extending generally normal thereto at a position permitting a minor portion of said body portion to extend therebeyond; and a twist disc integrally formed with and detachable from said shank portion to encase said minor portion including said distal end and protect both the device and its operator prior to use. The proximal end of the shank is provided with a circular notch for coaction with the jaws of a conventional dental contra-angle or standard straight handpiece for secured use.

A principal object of the present invention is to provide means and methods to facilitate the administration of a local anesthetic during medical and dental procedures to obtain quick and painless anesthesia without damaging surrounding tissue or bone structure or traumatizing the patient's central nervous system.

Still another object of the present invention is to provide a disposable intraosseous entry device in which a rotating beveled blade compresses adjacent bone structure without erosion to create a temporary port of ingress for the administration of an injectable local anesthetic to the temporarily accessed site.

A further object of the present invention is to provide an intraosseous entry device having a detachable twist disc operatively associated with the cutting edge thereof to protect both the blade and the operator prior to use and readily removable to expose the blade for use when the device is in the ready position.

These and still further objects as shall hereinafter appear are fulfilled by the present invention in a remarkably unexpected fashion as can be readily discerned from the following detailed description of an exemplary embodiment thereof, especially when read in conjunction with the accompanying drawing in which like parts bear like numerals throughout the several views

BRIEF DESCRIPTION OF THE DRAWINGS

In the drawings:
FIG. 1 is an isometric drawing illustrating intraosseous anesthesia;
FIG. 2 is an exploded isometric view of an intraosseous entry device embodying the present invention;
FIG. 3 is a cross-section taken on line 3-3 of FIG. 2; and
FIG. 4 is a plan view of a production tree containing a plurality of intraosseous entry devices;
FIG. 5 is an isometric view of a conventional dental handpiece having an entry device mounted therein for use in accordance with the present invention; and

FIG. 6 is an enlarged fragmentary view of the beveled face in a device embodying the present invention.

DESCRIPTION OF THE PREFERRED EMBODIMENTS

The present invention relates generally to means and methods to facilitate the administration of local anesthesia during medical and dental procedures and more particularly to an intraosseous entry device, identified in the drawing by the general reference 10, in which a rotating and strategically beveled blade 11 functions to displace and compress adjacent bone structure without erosion or damage to the bone structure to create a temporary port of ingress for the delivery of a local anesthetic to the temporarily accessed site.

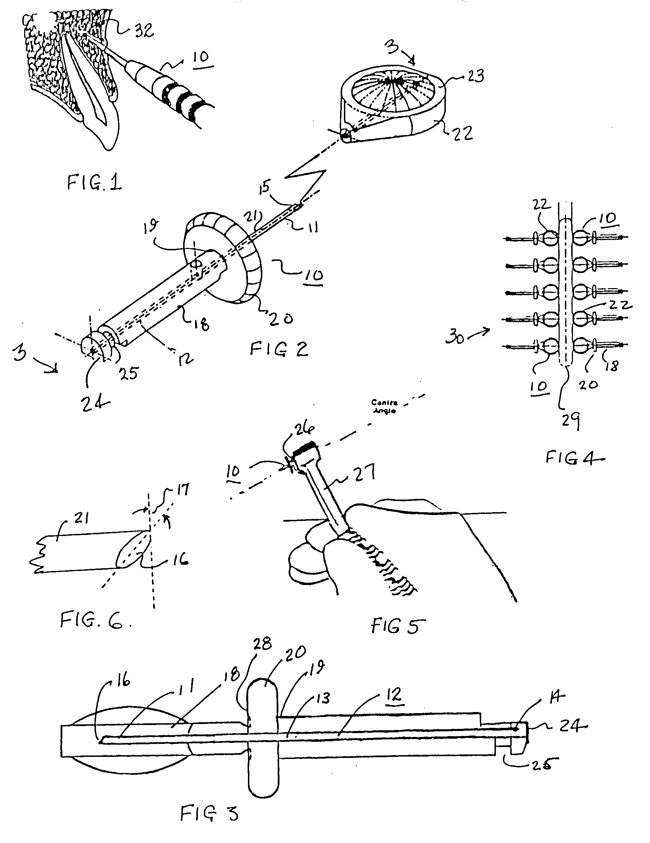

Referring to FIGS. 2 and 3 in which the device 10 is substantially enlarged for ease of description, each device 10 comprises an elongated solid cylindrical metal member 12 having an elongated body portion 13 extending between a proximal end 14 and a distal end 15. Distal end 15 is provided with a strategically beveled face 16 in a manner and for a purpose to be hereinafter described. Member 12 is preferably made out of stainless or surgical steel or a like non-corrodible alloy.

A tubular plastic shank member 18 is disposed in circumscription about body portion 13 and extends from proximal end 14 of member 12 approximately two-thirds the length of body portion 13 into engagement with a circular barrier member 20 which is integrally formed and circumscribed with shank member 18 and extending generally normal therefrom at a position permitting a minor portion 21 of said body portion 13 to extend therebeyond into a twist disc 22 integrally formed with and detachably secured to said barrier member 20. Twist disc 22 fully encases the minor portion 21 of said body portion 13, including beveled face 16 which with minor portion 21 comprises blade 11. As will appear twist disc 22 protects both the blade 11 and the user of device 10 from accidental encounter before use is desired or required.

Adjacent the proximal end 24 of shank 18 in slight spaced relationship thereto, a circular notch 25 is defined which, as will be hereinafter described, operatively coacts with the clamping jaws 26 of a conventional rotary driving means 27, such for example, as the dentistal contra-angle handpiece as shown in FIG.5.

In the preferred embodiment of the present invention, as shown in FIGS. 3 and 6, intraosseous entry device 10 includes at the lead or distal end 15 of solid cylindrical member 12 a beveled face 16 cut thereacross (also referred to as "tip 16"). The angle 17 of the face 16 relative to the longitudinal axis of member 12, is from about 25° to about 45° and preferrably between about 30° to about 35° for maximum compaction. Tip 16 is secured in a fixed position by the coaction of shank 18 upon cylindrical member 12 which, as described, is an extension of blade 11. Rounded barrier 20 circumscribes shank 18 adjacent metal tip 16. Shank 18 includes notched means 25 to facilitate the mounting of device 10 into the constrictive jaws of securing means 26 of a rotary device such as drill 27.

When assembled, intraosseous entry device 10 provides means for producing a temporary access or ingress port through bone structure with minimal thermal and mechanical trauma to the bone so accessed. In a preferred practice of the invention, device 10 is designed to enter and pass through, but not to remove particles from, intervening bone adjacent the preselected site for delivery of local anesthesia. As such, device 10 should not be confused with conventional material removers and/or eroder's such as reamers and burrs which grind off pieces of bone and inevitably traumatize adjacent soft tissues and frequently create an oversized receptor.

Reverting to FIGS. 2 and 3, the intraosseous entry device 10 shown therein comprises, interalia, a cylindrical shank 18. The rear or proximal end 24 of shank 18 has formed adjacent thereto a suitable recess 25 which, in coaction with the clamping jaws 26 of a conventional dental handpiece 27, secures device 10 therein for driven rotation in response to the driving rotation of handpiece 27 which is suitably powered in the conventional fashion. Adjacent the distal end 19 of the shank 18, enlarged Rounded barrier 20 obtains an interference fit with a protective twist disc 22 that is, there is a frangible interface therebetween. The surface 28 of Rounded barrier 20, when device 10 is in use, abuts the nose of the dental handpiece 27 and prevents handpiece 27 from becoming contaminated by fluids emanating from the patient's mouth. The protective twist disc 22 is also formed of a relatively hard medical-grade plastic material and has a roughened outer surface 23 to facilitate gripping thereof. Barrier 20 also functions as a detent or stoplimit to the depth of penetration obtained by beveled blade 11 when device 10 is used in accordance herewith.

In manufacture, shank 18 is preferably molded from a medical-grade plastic material and metal cylinder 12 will preferably be fixed within shank 18 during the molding of shank 18. As shown in FIG. 2, the proximal end 14 of member 12 which is secured within shank 18 is preferably disposed to insure that member 12 will not rotate relative to the shank 18 during use of device 10. As previously explained, it is important that the rotating blade 11 is rigidly fixed in shank 18 and turns therewith in response to the driving action of handpiece 27. It has been found that the provision of burrs or other roughening on the perimeter of member 12 at and adjacent proximal end 14 will enhance the anchoring of member 12 relative to the shank member 18.

In one practice of the present invention, a substantial portion of device 10 can be formed as a single molded unit which includes shank 18, rounded barrier 20, and protective twist disc 22. All of the plastic portions are preferably formed of medical-grade plastic. Cylindrical member 12 can also be disposed in the mold cavity before introducing the plastic thereinto. Preferably, device 10 will be cast in multiples in the shape of trees 30 extending from a central bar 29 as shown in FIG. 4. Such a production scheme substantially reduces unit manufacturing costs and allows the preferred disposability of device 10 after use to become economically feasible.

Device 10 is mounted for use with a suitable rotary handpiece 27 by gripping protective twist disc 22 and inserting the proximal end 24 of shank member 18 into the jaw 26 of the suitable handpiece 27. Once device 10 is firmly seated in handpiece 27, as by closing jaws 26 into circular notch 25 circumscribing shank 18, protective twist disc 18 may be removed by gripping surface 23 and rotating disc 18 until disc 18 is detached from barrier 20 and sterile metal tip 16 is exposed. As practiced, bevelled face 16 is maintained sterile until the moment of use.

Rounded barrier 20 is also useful in facilitating removal of device 10 from the dental handpiece 27 for disposal after use. Post-use disposal of contaminated device 10 is preferred because it eliminates the dangers associated with attempting to recap blade 11 with disc 22 after use and avoids operator contact with potentially toxic body fluids which may be present on the used metal tip 16 from patient effluvia. Such care is particularly important in an era when the exposure of medical personnel to aids, hepatitis and like virulent diseases is of grave concern. Such exposure can occur if such personnel have an open wound such as when a technician pricks a finger while attempting to recover blade 11. An important aspect of the device hereof and particularly the design of protective twist disc 22 is that the metal tip 16 cannot be recapped without extreme difficulty whereupon entry device 10 cannot accidentally be reused.

As previously described, rounded barrier 20 creates a detent or stop limit for the depth of penetration obtained by beveled blade 11 when device 10 is used to obtain intraosseous entry. The desired length of minor portion 21 of member 12, including beveled face 16, is preferably about 8mm which, by the limiting action of rounded barrier 20 becomes the maximum depth of penetration by blade 11.

When device 10 is manufactured as previously described, that is with a plurality of devices 10 a cast or molded in a oneshot sealed environment extending outwardly from a central bar 29 to form tree 30, the operator has a guarantee that a given device 10 has not been used at the time it is snapped from the tree. Furthermore, central bar 29 can be encoded if desired and using known procedures, which will permit both quality and quantity control of individual lots as well as a convenient means for identifying individual trees 30 if such is required by the environmental protection authorities who regulate the movement of biohazardous wastes from the surgical sites to regulated disposal sites.

In use, the practitioner will first select the site for injection based on the remedial or corrective procedure to be followed. The practioner will then disinfect and topically anesthetize the attached gingiva which covers the chosen entry site. The placement of the bevel of a standard gauge needle against the attached gingiva followed by the injection of a small amount (one or two drops) of local anesthetic solution therein until a slight blanching of the tissues is observed will anesthetize the attached gingiva and periosteum sufficiently for the comfortable use of device 10.

Next, one entry device 10 is detached from its associated control bar 29 and mounted into the jaws 26 of a suitable handpiece 27 such as a contra-angle or straight rotary drill and secured as previously described. Next protective twist disc 22 is turned and removed to expose minor portion 21 and bevel face 16 which form blade 11. The entry device 10 is then positioned so that the tip of beveled edge 16 is disposed against and shank 18 is perpendicular to the previously prepared surgical site. Next the rotary handpiece 27 is activated in the usual fashion while the practitioner gently presses the entry device 10 toward and into the bone disposed beneath the gingiva. Full entry is accomplished and will be recognized when there is a sudden give or lack of resistance to the forward movement of rotating blade 11. Entry device 10 can now be withdrawn from the temporary ingress port 32 it has created.

A standard 27 gauge needle syringe containing a local anesthetic of choice (such as Lignocaine, Mepivicaine, Prilocaine with Felypressin, Prilocaine and Lignocaine with Adrenalin and the like) can now be easily inserted into the ingress port 32, the required amount (one or two drops) of local anesthetic delivered to the site, and the syringe withdrawn.

From the foregoing, it is apparent that device 10 permits the ready entry into the cancellous bone through the attached gingiva, periosteum and cortical plate of bone to form a temporary ingress port therethrough. After withdrawal of device 10 a conventional anesthetic syringe needle is employed to inject a controlled amount of anesthetic solution through the ingress port thus created directly to the specific site selected for the intended procedure. Only a small amount of anesthetic solution is required because of its propitious site of delivery, which anesthetic acts instantly to literally guarantee profound pulpal anesthesia and avoid the ballooning of the soft tissues. Further, practice hereof does not leave the patient numb for hours after the procedure and avoids the risk of hematoma or trismus. The means and methods herein described and illustrated permits both sides of the mandible to be treated at the same session, eliminates palatal injections for extractions and is virtually painless.

From the foregoing it can be readily ascertained that means and methods for facilitating the delivery of local anesthesia have been herein described and illustrated which fulfill all of the foregoing objectives in a remarkably unexpected fashion. It is, of course, understood that such modifications, alterations and adaptations, as may readily occur to the artisan confronted with this disclosure, are intended within the spirit of the present invention which is limited solely by the scope of the claims appended hereto.

**Claims**

1. An intraosseous entry device for use with a rotary driver characterised by comprising:

   an elongated cylindrical member having a proximal end and a distal end, said distal end having a beveled face defined thereon;

   a shank member disposed in circumscription about said cylindrical member and having means defined therein for cooperative engagement by and coaction with a rotary driver to secure said shank member therein for driven rotation in response thereto, said shank member further having a rounded barrier member disposed thereabout adjacent and in spaced relationship to said distal end of said cylindrical member and a protective twist disc attached to said barrier member and detachable therefrom to respectively encase and expose said beveled face of said cylindrical member.

2. A device according to Claim 1 characterised in that said beveled face is disposed relative to the longitudinal axis of said cylindrical member to define an angle of from about 25° to about 45°.

3. A device according to Claim 2 characterised in that said defined angle is from about 30° to 40°.

4. A device according to any one of claims 1 to 3 characterised in that said shank member is formed of molded medical grade plastic and a portion of said cylindrical member is embedded therein along the longitudinal central axis thereof.

5. A device according to any other preceding claim characterised in that said protective twist disc is integrally formed with said rounded barrier member, said rounded barrier member being positioned relative to said shank member to abut against a rotary driver when said shank member is operatively inserted therein.

6. A device according to Claim 5 characterised in that said protective twist disc and said shank member coact to encase said elongated cylindrical member, said protective twist disc being removable to expose said beveled face.

7. A method of delivering local anesthesia to a preselected injection site disposed beneath cancellous bone, said method being characterised by comprising creating a temporary ingress port to said injection site through cancellous bone without removing any bone or tissue therefrom; inserting the needle of a syringe containing a preselected amount of anesthesia into said temporary ingress port in juxtaposition with said injection site; ejecting a small but effect amount of said anesthesia from said syringe onto said injection site; and withdrawing said needle from said temporary ingress port.

8. A method according to Claim 7 in which said temporary ingress port is created by preselecting a line of entry to said preselected injection site; displacing and compacting cancellous bone outwardly from said preselected line of entry in an amount sufficient to permit the passage of hypodermic syringe needle into and out of said port.

FIG.1

FIG 2

FIG 4

FIG. 6.

FIG 5

FIG 3

European Patent Office

**PARTIAL EUROPEAN SEARCH REPORT**
which under Rule 45 of the European Patent Convention shall be considered, for the purposes of subsequent proceedings, as the European search report

Application number

EP 91302806

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | US-A-4157714 (FOLTZ et al.) <br> * column 1, line 51 - column 2, line 12 ; column 3, lines 28-39; figures 1,12,13, 21 * | 1 | A61B17/16 <br> A61M19/00 |
| A | US-A-3893445 (HOFSESS) <br> * the whole document * | 1 | |
| A | US-A-4811736 (GRIGGS et al.) <br> * the whole document * | 1 | |

TECHNICAL FIELDS
SEARCHED (Int. Cl.5)

A61B
A61M
A61C

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-6
Claims searched incompletely: —
Claims not searched: 7,8
Reason for the limitation of the search:

article 52 (4)   EPC

method for treatment of the human body by surgery

| Place of search | Date of completion of the search | Examiner |
|---|---|---|
| BERLIN | 29.10.1991 | ROLAND, A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document